# EUROPÄISCHE PATENTSCHRIFT

(11) **EP 0 678 512 B1**
(45) Veröffentlichungstag und Bekanntmachung des Hinweises auf die Patenterteilung: **16.02.2000**
(21) Anmeldenummer: 95105702.5
(22) Anmeldetag: 15.04.1995
(51) Int. Cl.: C07D 301/00, C07D 303/02, C07D 303/24, C08G 59/14

(54) **Verfahren zur selektiven Hydrierung von aromatischen Gruppen in Gegenwart von Epoxygruppen**
Process for the selective hydrogenation of aromatic groups in presence of epoxy groups
Procédé pour l'hydrogénation sélective de groupes aromatiques en présence de groupes époxy

(30) Priorität: 22.04.1994 DE 4414089
(43) Veröffentlichungstag der Anmeldung: 25.10.1995
(73) Patentinhaber: BASF Aktiengesellschaft, 67063 Ludwigshafen (DE)
(72) Erfinder: Wettling, Thomas, Dr., D-67117 Limburgerhof (DE); Schuster, Ludwig, Dr., D-67117 Limburgerhof (DE); Henkelmann, Jochem, Dr., D-68165 Mannheim (DE)

(56) Entgegenhaltungen:
- EP-A- 0 258 789
- EP-A- 0 402 743
- EP-A- 0 545 154
- US-A- 3 336 241
- TETRAHEDRON LETTERS, Bd.36, Nr.6, 6. Februar 1995, OXFORD GB Seiten 885 - 888 F. FACHE ET AL. 'A catalytic stereo- and chemo-selective method for the reduction of aromatics'

## Beschreibung

Die vorliegende Erfindung betrifft ein verbessertes Verfahren zur selektiven Hydrierung von aromatischen Gruppen organischer Moleküle, die mindestens eine aromatische Gruppe und eine Epoxygruppe tragen, mit Wasserstoff in Gegenwart eines rutheniumhaltigen Katalysators.

Die EP-A-545 154 beschreibt ein Verfahren zur Herstellung von Diglycidyloligoethern durch katalytische Hydrierung, wobei man Rutheniumoxidhydrat als Katalysator verwendet und die Hydrierung bei 20 bis 60°C und einem Wasserstoffdruck von 100 bis 400 bar vornimmt.

Die US-A 3 336 241 lehrt die Hydrierung von aromatischen Epoxyverbindungen mit Rhodium- und Rutheniumkatalysatoren. Die Aktivität der Katalysatoren nimmt nach einer Hydrierung so stark ab, daß in einem technischen Verfahren der Katalysator nach jeder Hydrierung gewechselt werden muß. Die US-A 3 966 636 lehrt ein Verfahren zur Regenerierung von Rhodium- und Rutheniumkatalysatoren, die bei der Hydrierung von 2,2-Di-[p-glycidoxi-phenyl]-propan desaktiviert worden sind.

Es stellte sich die Aufgabe, ein selektives Hydrierverfahren bereitzustellen, in welchem die eingesetzten Katalysatoren ohne Aufarbeitung mehrfach für Hydrierungen eingesetzt werden können.

Die DE-A 36 29 632 und die DE-A 39 19 228 lehren die selektive Hydrierung von Di-[glycidoxi-phenyl]-methan bzw. von 2,2-Di-[p-glycidoxi-phenyl]-propan an Rutheniumoxidhydrat. Auch nach dieser Lehre empfiehlt es sich, die Katalysatoren nach jeder Hydrierung zu regenerieren. Dabei treten Schwierigkeiten bei der Abtrennung des feinverteilten Katalysators auf, die überwiegend durch Verwendung von Filtrationshilfsmitteln gelöst werden können. Zur Aufarbeitung der Katalysatoren müssen diese jedoch von den Filtrationshilfsmitteln getrennt werden.

Es bestand daher weiterhin die Aufgabe, ein Verfahren bereitzustellen, das eine leichte Abtrennbarkeit des Katalysators Rutheniumoxidhydrat vom Hydrieraustrag erlaubt.

Demgemäß wurde das eingangs definierte Verfahren gefunden, das dadurch gekennzeichnet ist, daß man die Hydrierung in Gegenwart von 0,2 bis 10 Gew.-% Wasser, bezogen auf den Reaktionsansatz, vornimmt.

Als Ausgangsverbindungen kommen alle solche organische Moleküle in Betracht, die mindestens eine aromatische Gruppe und eine Epoxygruppe tragen. Dabei kann es sich um monomere, oligomere oder polymere Verbindungen handeln. Als Ausgangsverbindungen für das erfindungsgemäße Verfahren sind folgende Substanzklassen und Stoffe zu nennen:
- Reaktionsprodukte aus Bisphenol A bzw. Bisphenol F und Epichlorhydrin
   Bisphenol A bzw. Bisphenol F und Epichlorhydrin können mit Basen in bekannter Weise (z.B. Ullmann's Encyclopedia of Industrial Chemistry, 5th Edition, VCH (1987) Vol. A9, S. 547) zu Glycidylethern der allgemeinen Formel I umgesetzt werden 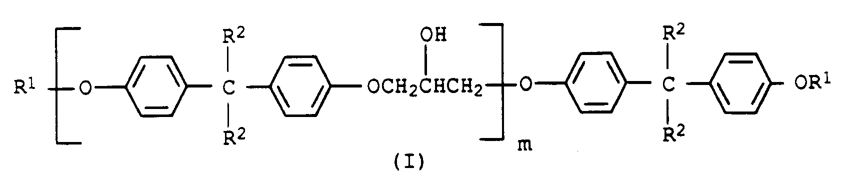 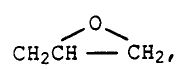 wobei R¹ für R² für Wasserstoff oder eine Methylgruppe und m für Null bis 40 steht.
- Phenol- und Kresolepoxynovolake
   Novolake der allgemeinen Formel II sind durch säurekatalysierte Reaktion von Phenol bzw. Kresol und Epoxidierung der Reaktionsprodukte erhältlich (s. z.B. Bis[4-(2,3-epoxypropoxy)phenyl]methan): 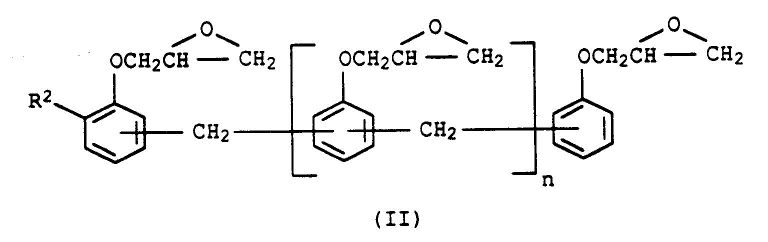 wobei R² für Wasserstoff oder eine Methylgruppe und n für 0 bis 40 steht.
- Glycidylether von Reaktionsprodukten aus Phenol und einem Aldehyd
   Durch säurekatalysierte Umsetzung von Phenol und Aldehyden und anschließende Epoxidierung mit Epichlorhydrin sind Glycidylether zugänglich, z.B. ist 1,1,2,2-Tetrakis-[4-(2,3-epoxypropoxy)phenyl]ethan aus Phenol und Glyoxal zugänglich.
- Aromatische Glycidylamine
   Beispielhaft sind die Triglycidylverbindung von p-Aminophenol, 1-(2,3-epoxypropoxy)-4-[N,N-bis(2,3-epoxypropyl)-amino]benzol, und die Tetraglycidylverbindung von Methylendiamin Bis{4-[N,N-bis(2,3-epoxypropyl)amino[phenyl}methan zu nennen.

Im einzelnen sind weiterhin zu nennen:

1,1,2,2-Tetrakis[4-(2,3-epoxypropoxy)phenyl]ethan, Tris[4-(2,3-epoxypropoxy)phenyl]methan-isomere, 2,5-Bis[(2,3-epoxypropoxy)phenyl]octahydro-4,7-methano-5H-inden.

Bevorzugte Ausgangsverbindungen sind Di-[p-glycidoxi-phenyl]-methan und 2,2-Di-[p-glycidoxi-phenyl]-propan und Oligomere dieser Verbindungen.

Als Katalysatoren kommen für das erfindungsgemäße Verfahren homogene und heterogene Rutheniumkatalysatoren in Betracht. Metalli-sches Ruthenium auf einem Kohle- oder Aluminiumoxidträger ist ebenso zu nennen wie auf Metalloberflächen aufgedampftes Ruthenium. Solche Katalysatoren sind im Handel oder durch an sich bekannte Methoden erhältlich. Bevorzugt wird jedoch Rutheniumoxidhydrat, das sowohl homogen wie auch an heterogene Träger gebunden eingesetzt werden kann. Man erhält diese Rutheniumverbindung, die der Formel Ru₂O₃ · xH₂O entspricht, wobei x Werte über 1 annehmen kann, als wasserfeuchten Niederschlag durch Umsetzung einer wäßrigen Lösung von Ruthenium-III-chloridhydrat RuCl₃ x 3H₂O mit Natronlauge und anschließendes Waschen mit Wasser zur Entfernung der Chloridionen. Die Menge des Katalysators liegt in der Regel bei 0,01 bis 1 Gew.-% Ruthenium, bezogen auf die zu hydrierende Ausgangsverbindung.

Da die erfindungsgemäßen Hydrierungen in vielen Fällen mit viskosen Produkten durchgeführt werden, kann es vorteilhaft sein, die Reaktion in einem Lösungsmittel auszuführen. Als Lösungsmittel sind Ether bevorzugt, z.B. Tetrahydrofuran, Dioxan, tert.-Butyl-methylether, Glykoldimethylether und Methoxypropanol. Die Menge des Lösungsmittels beträgt im allgemeinen 5 bis 80 Gew.-%, bezogen auf den Reaktionsansatz.

Weiterhin werden dem Reaktionsansatz 0,2 bis 10 Gew.-% Wasser, bezogen auf den Reaktionsansatz, zugesetzt. Während bei geringeren Mengen kein Effekt erkennbar ist, kommt es bei deutlich größeren Mengen in verstärktem Maße zur unerwünschten hydrolytischen Öffnung des Epoxidrings.

Die Hydrierung wird unter Druck ausgeführt, der in der Regel bei 100 bis 320 bar liegt. Die Reaktionstemperatur beträgt in der Regel 30 bis 80°C, bevorzugt 40 bis 70°C.

Die Reaktion kann diskontinuierlich und kontinuierlich ausgeführt werden. Dazu können die Ausgangsverbindungen, der Katalysator, Wasser und gegebenenfalls Lösungsmittel vermischt und in einem Reaktor mit Wasserstoff umgesetzt werden. Die Reaktion ist im allgemeinen nach 2 bis 10 Stunden beendet. Der Reaktionsansatz kann dann auf Normaldruck entspannt, von Katalysator beispielsweise durch Filtration abgetrennt und durch Destillation von allen flüchtigen Bestandteilen befreit werden. Der abgetrennte Katalysator kann, gewünschtenfalls nach Ergänzung durch frischen Katalysator, in das Hydrierverfahren zurückgeführt werden.

Das erfindungsgemäße Verfahren hat den Vorteil, daß der Katalysator nach einer Hydrierung noch so aktiv ist, daß er für weitere Hydrierungen eingesetzt werden kann. Es hat sich als zweckmäßig erwiesen, nach jeder Hydrierung vor der Rückführung des gebrauchten Katalysators einen kleinen Teil, z.B. ein Viertel, durch neuen Katalysator zu ersetzen, wodurch sich der für eine Katalysatorregenerierung erforderliche technische Aufwand beträchtlich vermindert.

Weiterhin erlaubt die vorliegende Erfindung bei Verwendung von Rutheniumoxidhydrat eine einfache Katalysatorabtrennung vom Produkt.

Die Verfahrensprodukte finden als lichtbeständige Anstrichmittel, Gießharze und Laminate Verwendung.

### Beispiele

### Beispiel 1 bis 4

In einem Autoklaven wurden 1000 g eines Bisglycidylethers eines Phenol-Formaldehyd-Kondensates (Bis[4-(2,3-epoxypropoxy)phenyl]-methan mit einem Epoxidäquivalentgewicht von 168), 40 g einer Rutheniumoxidhydratsuspension in Tetrahydrofuran THF mit einem Gehalt von 1 g Ruthenium (erhalten durch Umsetzung von RuCl₃ x 3H₂O mit Natronlauge bei pH 8 und Waschen des so erhaltenen Niederschlags mit Wasser und THF), Wasser und 960 g THF bei 50°C und einem Druck von 250 bar mit Wasserstoff hydriert. Die Wassermenge des Ansatzes sowie die Reaktionsdauer ist der folgenden Tabelle 1 zu entnehmen. Die Hydrierausträge wurden filtriert und waren farblos. Es wurden nach dem Abdestillieren flüchtiger Bestandteile 1010 bis 1030 g Produkt isoliert.

**Tabelle 1**

| Beispiel | Wassergehalt [Gew.-%] | Reaktionszeit [h] | Aromatengehalt Produkt | Epoxidäquivalentgewicht |
|---|---|---|---|---|
| 1 | 1,0 | 6,0 | 4 % | 188 |
| 2 | 2,5 | 4,0 | Spur | 191 |
| 3 | 5,3 | 3,0 | 0 | 197 |
| 4 | 7,5 | 2,5 | 0 | 213 |

Das Epoxidäquivalentgewicht wurde nach ASTM D 1652-88 bestimmt und gibt das mittlere Molekulargewicht des Verfahrensproduktes dividiert durch die mittlere Zahl an Epoxygruppen pro Molekül an. Der Wert ist somit ein Maß für die Selektivität der Hydrierreaktion.

### Beispiel 5 (Vergleich)

### Durchführung wie Beispiele 1 bis 4, jedoch ohne Wasserzugabe

Nach einer Reaktionsdauer von 8 h wurden 1010 g Produkt mit 3 % Aromatengehalt und einem Epoxidäquivalentgewicht von 177 isoliert.

Der Reaktionsaustrag war nach Filtration dunkel gefärbt und konnte nur durch Zugabe von Aktivkohle entfärbt werden.

Das erfindungsgemäße Verfahren erlaubt bei kürzerer Reaktionszeit die Herstellung reinerer Produkte. Weiterhin ist die vollständige Abtrennung des Katalysators vom Produkt technisch weniger aufwendig als im Vergleichsversuch.

### Beispiel 6

In einem Autoklaven wurden 1000 g des in Beispiel 1 beschriebenen Polyglycidylether, 40 g Rutheniumoxidhydrat-THF-Suspension mit einem Rutheniumgehalt von 1 g, 40 g Wasser und 920 g THF bei 250 bar 4 h bei 50°C bis 70°C mit Wasserstoff hydriert. Nach Entspannen auf Normaldruck ließ man den Katalysator 12 h absitzen. Von der überstehenden Lösung wurden 1400 g über ein Steigrohr entnommen. Der Reaktionsansatz wurde mit dem Bisglycidylether, Wasser, THF und Katalysator auf 2000 g aufgefüllt, wobei jedoch statt 1 g Ruthenium nur 0,25 g zugesetzt wurden (Gewichtsverhältnis von Polyglycidylether zu THF 10:9, Wassergehalt 2,6 Gew.-%). Bei drei nachfolgenden Hydrierungen wurden jeweils 2020 g Reaktionsaustrag entnommen und wie beschrieben ersetzt. Alle Reaktionsausträge waren nach Filtration farblos, praktisch aromatenfrei und besaßen Epoxidäguivalentgewichte von 180 bis 185.

Durch Zugabe von nur geringen Mengen frischen Katalysators nach jeder Hydrierung können mehrere Hydrierungen bei konstanter Produktqualität durchlaufen werden.

### Beispiel 7 (Vergleich, ohne Wasser)

1000 g des in Beispiel 1 charakterisierten Bisglycidylethers, 40 g der Rutheniumoxidhydrat-THF-Suspension mit einem Gehalt von 1 g Ruthenium und 900 g THF wurden 4 h bei 50°C bis 70°C und 250 bar mit Wasserstoff hydriert. Man ließ den Katalysator 12 h absitzen und entnahm wie in Beispiel 6 beschrieben über ein Steigrohr 1400 g der überstehenden Lösung, zentrifugierte mitgerissenen Katalysator ab - dabei gelang keine vollständige Abtrennung - und führte den so isolierten Katalysator mit den oben angegebenen Mengen an Polyglycidylether und THF in den Reaktor zurück. Eine erneute Hydrierung kam nach nur geringer Wasserstoffaufnahme zum Stillstand.

Der Katalysator war nach einer Hydrierung desaktiviert.

### Beispiel 8 (Kontinuierliche Fahrweise)

Ein Bisglycidylether, wie er in Beispiel 1 charakterisiert worden ist, wurde mit Tetrahydrofuran im Gewichtsverhältnis von 1:0,96 in Gegenwart von 0,1 Gew.-% Ruthenium, bezogen auf den Reaktionsansatz, das in Form der in Beispiel 1 beschriebenen Rutheniumoxidhydratsuspension eingesetzt wurde, sowie von 0,5 Gew.-% Wasser, bezogen auf den Reaktionsansatz, bei 50 bis 70°C und einem Wasserstoffdruck von 250 bar bei einer mittleren Verweilzeit von 10 h kontinuierlich hydriert. Der Reaktionsaustrag wurde vom Katalysator abgetrennt und die flüchtigen Komponenten wurden abdestilliert. Das verbleibende Produkt war farblos (Aromatengehalt 6,7 bis 9,4 %, Epoxidäquivalentwert 179 bis 187). Der abgetrennte Katalysator wurde mit 0,03 Gew.-%, bezogen auf den Reaktionsansatz, frischem Ruthenium (in Form von Rutheniumoxidhydratsuspension) angereichert und mit den Ausgangsverbindungen in die Reaktion zurückgeführt.

## Patentansprüche

1. Verfahren zur selektiven Hydrierung von aromatischen Gruppen organischer Moleküle, die mindestens eine aromatische Gruppe und eine Epoxygruppe tragen, mit Wasserstoff in Gegenwart eines rutheniumhaltigen Katalysators, dadurch gekennzeichnet, daß man die Hydrierung in Gegenwart von 0,2 bis 10 Gew.-% Wasser, bezogen auf den Reaktionsansatz, vornimmt.

2. Verfahren nach Anspruch 1, dadurch gekennzeichnet, daß man als Katalysator Rutheniumoxidhydrat verwendet.

3. Verfahren nach Anspruch 1 oder 2, dadurch gekennzeichnet, daß man Di-[p-glycidoxiphenyl]-methan oder 2,2-Di-[p-glycidoxi-phenyl]-propan hydriert.

4. Verfahren nach den Ansprüchen 1 bis 3, dadurch gekennzeichnet, daß man die Hydrierung bei 40 bis 70°C vornimmt.

## Claims

1. A process for the selective hydrogenation of aromatic groups in organic molecules having at least one aromatic group and one epoxy group with hydrogen in the presence of a ruthenium-containing catalyst, wherein the hydrogenation is carried out in the presence of from 0.2 to 10% by weight of water based on the reaction mixture.

2. A process as claimed in claim 1, wherein ruthenium oxide hydrate is used as catalyst.

3. A process as claimed in claim 1 or 2, wherein di[p-glycidoxyphenyl]methane or 2,2-di[p-glycidoxyphenyl]propane is hydrogenated.

4. A process as claimed in claims 1 to 3, wherein the hydrogenation is carried out at from 40 to 70°C.

## Revendications

1. Procédé d'hydrogénation sélective des groupements aromatiques de molécules organiques portant au moins un groupement aromatique et un groupement époxy, avec de l'hydrogène en présence d'un catalyseur contenant du ruthénium, caractérisé en ce que l'on effectue l'hydrogénation en présence de 0,2-10% en poids d'eau, par rapport à la charge réactionnelle.

2. Procédé selon la revendication 1, caractérisé en ce que l'on utilise un oxyde de ruthénium hydraté en tant que catalyseur.

3. Procédé selon la revendication 1 ou 2, caractérisé en ce que l'on hydrogène le di[p-glycidoxyphényl]méthane ou le 2,2-di[p-glycidoxyphényl]-propane.

4. Procédé selon l'une quelconque des revendications 1 à 3, caractérisé en ce que l'on effectue l'hydrogénation à 40-70°C.
